# EUROPEAN PATENT APPLICATION

(11) **EP 4 368 111 A1**
(43) Date of publication of application: **15.05.2024**
(21) Application number: 23205158.1
(22) Date of filing: 23.10.2023
(51) Int. Cl.: A61B 6/04, A61G 13/12

(54) **METHODS AND SYSTEMS FOR A MULTI-POSITIONER HOLDER**

(30) Priority: 10.11.2022 US 202218054445
(71) Applicant: GE Precision Healthcare LLC, Waukesha, WI 53188 (US)
(72) Inventor: LEWIS, Chelsey Amanda, Waukesha, 53188 (US); DELONG SAMALIK, Michelle Marie Severino, Waukesha, 53188 (US); EVANGELIST, Matthew J., Waukesha, 53188 (US); SMITH, Chad A., Waukesha, 53188 (US); NETT, Brian E., Waukesha, 53188 (US)
(74) Representative: Fennell, Gareth Charles

(57) **Abstract**

Various systems are provided for a support assembly for a medical imaging system. In one example, a system comprising, a support assembly for use with an imaging system, the support assembly comprising, a cradle including a base and opposing sidewalls, and a plurality of pads shaped to seat against the base within an opening formed between the opposing sidewalls, where each pad of the plurality of pads supports a head or extremity of a subject to be imaged at a different angle relative to the base.

## Description

### TECHNICAL FIELD

Embodiments of the subject matter disclosed herein relate to methods and systems for supporting a head or extremity of a patient body.

### BACKGROUND

A support system for a patient head or extremity may be used in a variety of environments. Support systems capable of providing a range of angles may be used during imaging scans to support and position a patient head or limb through a range of discrete angles. The ability to tilt a patient's head or limb and hold that position throughout an imaging scan is very important for image quality. In one example, adjustable head holders may be used in a medical setting to select and maintain the position of a patient's head to avoid imaging dental implants and other devices that may create image artifacts during a medical imaging procedure. In addition, tilting a patient's head may allow a reduction in radiation dose to a sensitive anatomy, such as the eyes by placing them outside of the x-ray radiation beam. An adjustable head holder allows an imaging technologist or operator to position a patient's head in such a way that without the device, the patient would not be able to hold his/her head in a specific orientation during a scan. Other patients who would benefit from the use of an adjustable head holder can be subject to involuntary movements or may be combative. Having a mechanism for selecting and maintaining the position of the head ensures that patients subject to the above conditions cannot change the tilting angle of the adjustable head holder themselves. A further benefit to the adjustable head holder is that some of the positions in which the patient's head is positioned may be uncomfortable without support from the support system.

### BRIEF DESCRIPTION

In one example, a system comprising, a cradle including opposing sidewalls; and a plurality of pads shaped to seat against the cradle within a clearance formed between the opposing sidewalls, where each pad of the plurality of pads supports a head or appendage of a subject to be imaged at a different angle relative to the cradle. In this way, the head or appendage of the subject may be supported at a variety of discrete angles, and the angles easily adjusted by the operator during a procedure.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present disclosure will be better understood from reading the following description of non-limiting embodiments, with reference to the attached drawings, wherein below:
FIG. 1A shows a pictorial view of an imaging system, in accordance with one or more embodiments of the present disclosure;
FIG. 1B shows a view of a patient table having a mount for a multi-positioner holder, in accordance with one or more embodiments of the present disclosure;
FIG. 2 shows a block schematic diagram of an exemplary imaging system, in accordance with one or more embodiments of the present disclosure;
FIG. 3A shows a first view of an example of a multi-positioner holder, in accordance with one or more embodiments of the present disclosure;
FIG. 3B shows a second view of the example of the multi-positioner holder, in accordance with one or more embodiments of the present disclosure;
FIG. 3C shows a third view of the example of the multi-positioner holder, in accordance with one or more embodiments of the present disclosure;
FIG. 3D shows a fourth view of the example multi-positioner holder, in accordance with one or more embodiments of the present disclosure;
FIG. 4A and FIG. 4B show front and rear perspective views, respectively, of a first example of a pad for the exemplary multi-positioner holder, in accordance with one or more embodiments of the present disclosure;
FIG. 5A and FIG. 5B show front and rear perspective views, respectively, of a second example of a pad for the exemplary multi-positioner holder, in accordance with one or more embodiments of the present disclosure;
FIG. 6A and FIG. 6B show front perspective and cross section views, respectively, of a third example of a pad for the exemplary multi-positioner holder, in accordance with one or more embodiments of the present disclosure;
FIG. 7A, FIG. 7B, and FIG. 7C show an example of a first position, a second position and a third position, respectively, for a multi-positioner holder, in accordance with one or more embodiments of the present disclosure;
FIG. 8 shows a front perspective view of a first example configuration for the plurality of pads for the exemplary multi-positioner holder, in accordance with one or more embodiments of the present disclosure;
FIGS. 9A and FIG. 9B show rear and front perspective views, respectively, of a second example configuration for the plurality of pads for the exemplary multi-positioner holder, in accordance with one or more embodiments of the present disclosure;
FIG. 10A and FIG. 10B shows a rear perspective view of a third example configuration for the plurality of pads for the exemplary multi-positioner holder, in accordance with one or more embodiments of the present disclosure;

### DETAILED DESCRIPTION

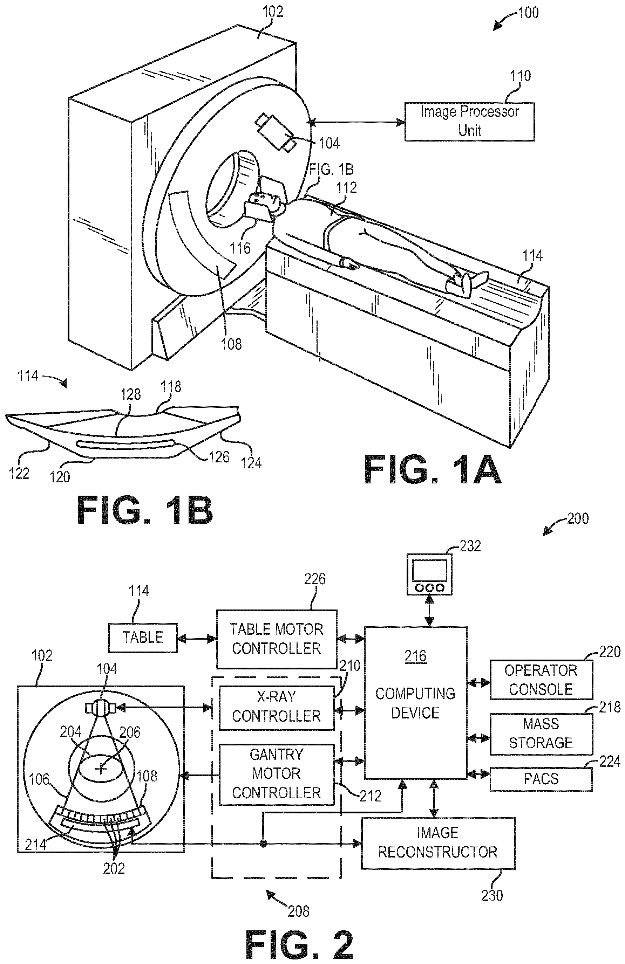

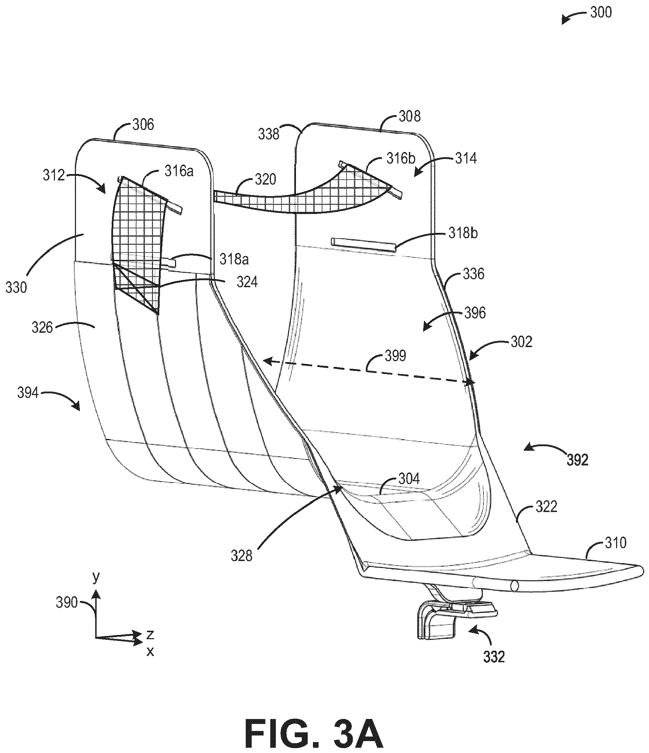

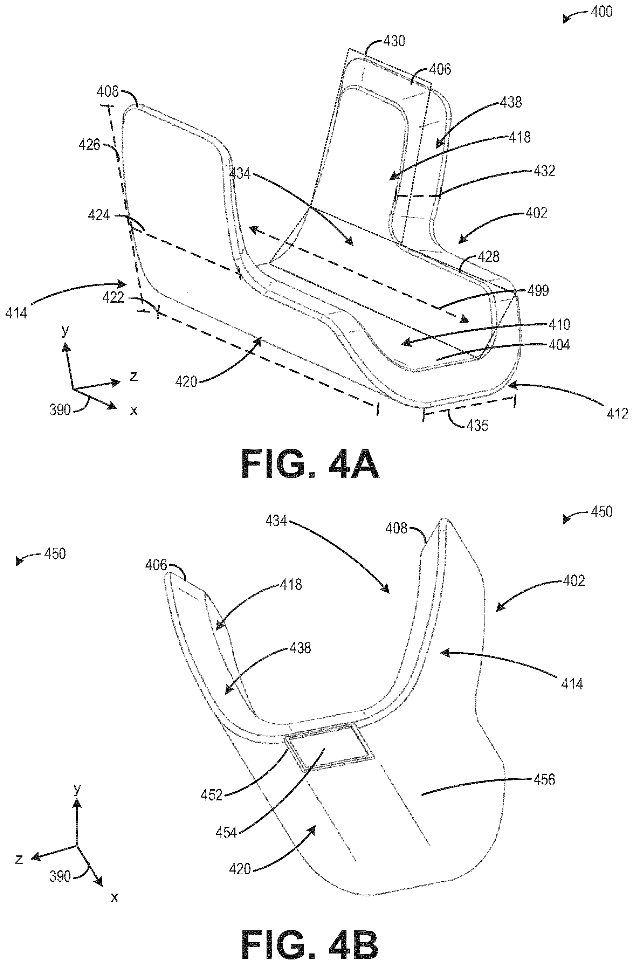

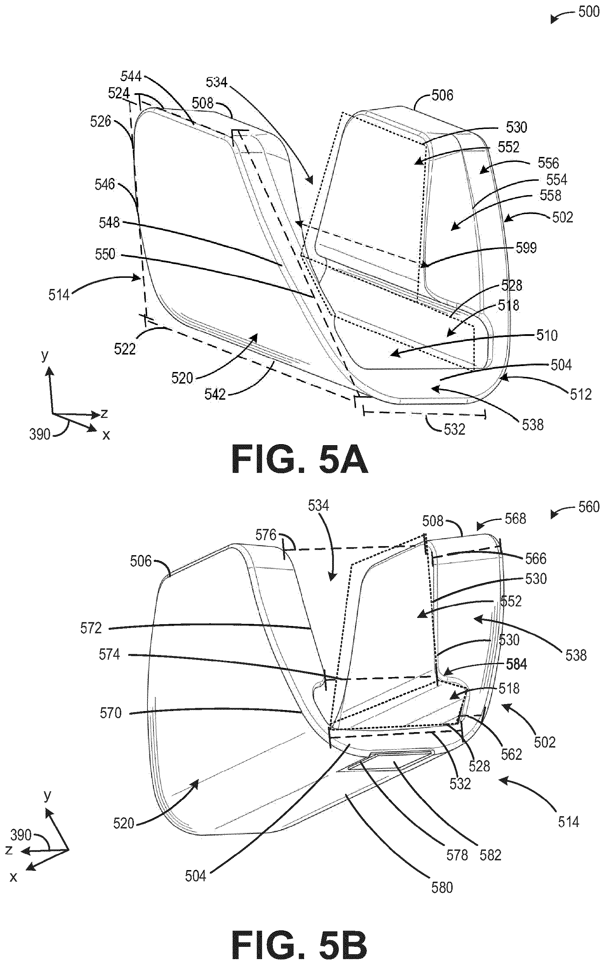

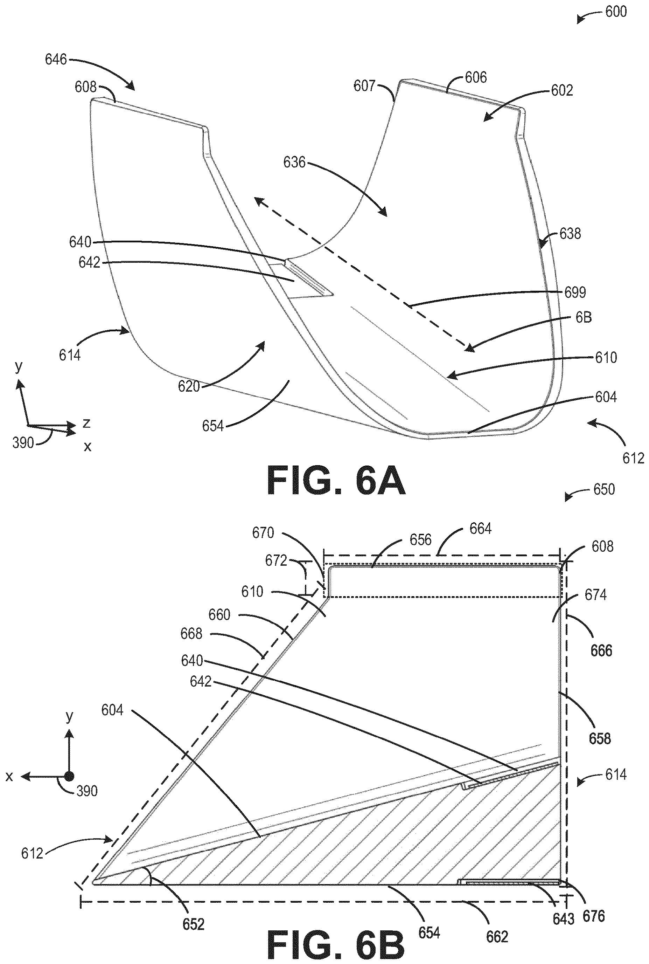

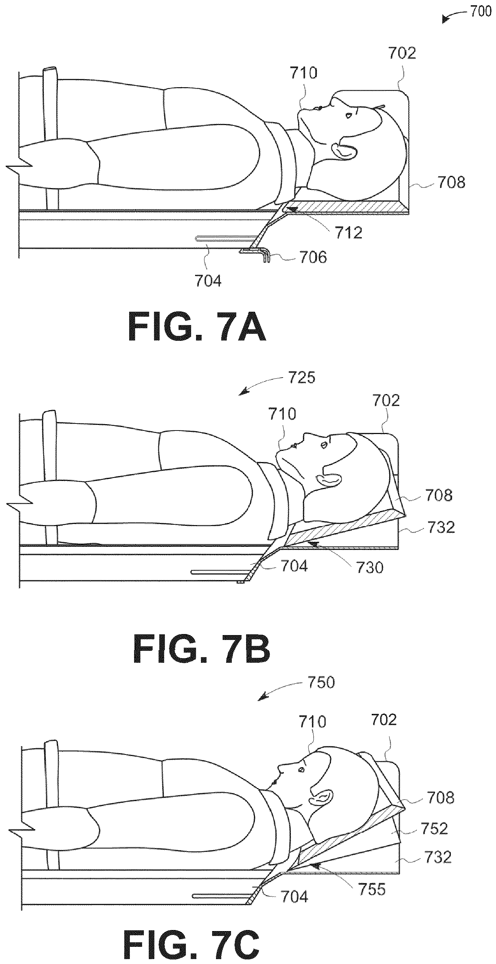

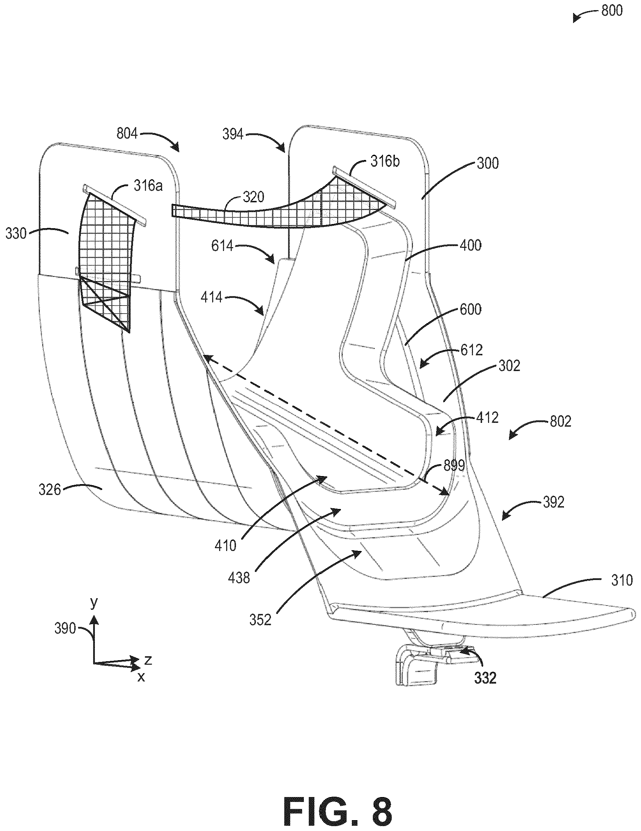

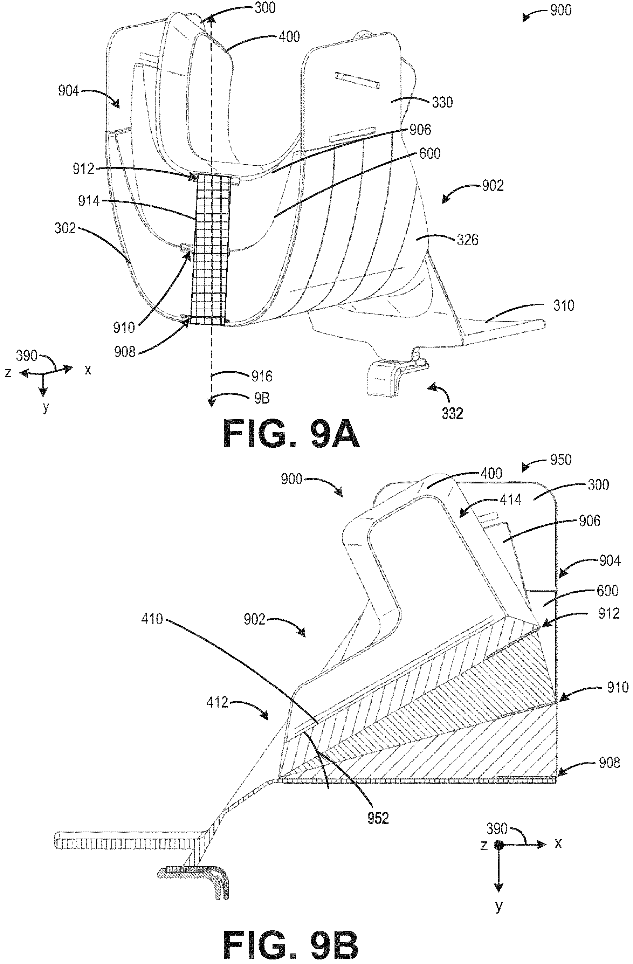

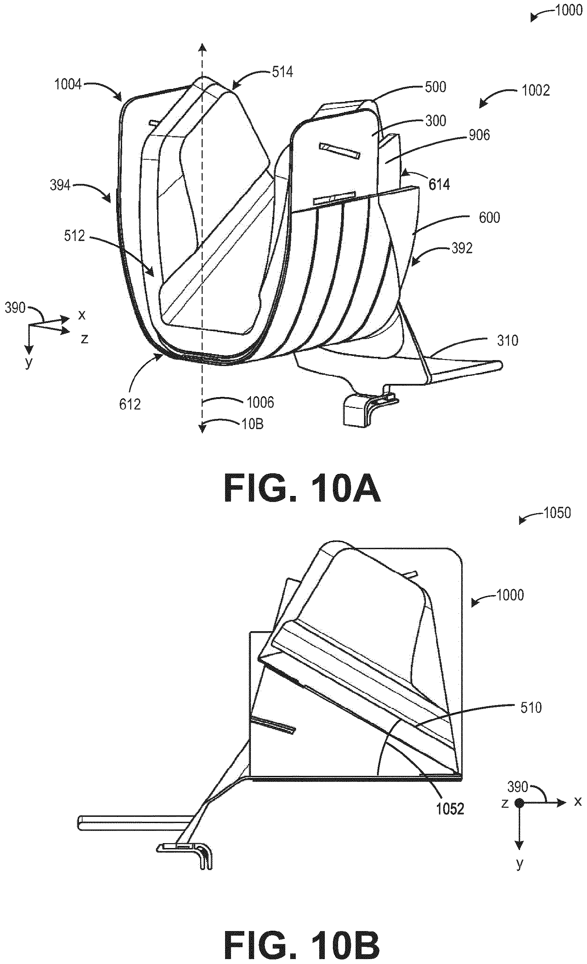

The following description relates to examples of a support assembly for a medical imaging system. In one example, the system is a multi-positioner holder for an imaging system, such as a computerized tomography (CT) imaging system illustrated in FIGS. 1A-2. A first example of the multi-positioner holder comprises a cradle including opposing sidewalls for supporting a subject to be imaged, as shown in FIGS. 3A-3D. The multi-positioner holder may be used with a plurality of pads shaped to seat against the cradle within a clearance formed between the opposing sidewalls, where each pad of the plurality of pads supports a head or extremity of the subject to be imaged. In one example, the plurality of pads may include a pad shaped to support the head at a first angle. Such an example is shown in FIG. 4A and FIG. 4B. In one example, the plurality of pads may include a pad shaped to support an extremity, e.g., foot or hand, at the first angle. Such an example is shown in FIG. 5A and FIG. 5B. The exemplary head and extremity pads may be used singly, or one of the head and extremity pads may be used in-combination with a pad shaped at a different, second angle. An example angled positioning pad is illustrated in FIG. 6A-6B. The pads may be positioned within the cradle in various configurations, such as singly and in-combination. FIG. 7A, 7B, and 7C show an example of a first position, a second position, and a third position, respectively, that may be achieved using various configurations of the plurality of pads positioned in the multi-positioner holder. A first example configuration for the plurality of pads for the exemplary multi-positioner holder is illustrated in FIG. 8. FIGS. 9A-9B illustrate front and cross-section views, respectively, of a second example configuration for the plurality of pads for the exemplary multi-positioner holder. In some examples, the plurality of pads and multi-positioner holder may be used to produce positive or negative angles for imaging. FIG. 10A and FIG. 10B illustrate an example configuration for the plurality of pads positioned in the multi-positioner holder to produce a negative angle. FIG. 3A through FIG. 6B, and FIG. 8 through FIG. 10B are shown to scale, however, other relative dimensions may be used if desired.

In one example, the multi-positioner holder may assist a radiologist to position a head or other extremity of a subject (also referred to herein as a patient) during computed tomography x-ray examination. Previous examples include where a fixed head holder is used of an axial head scan in a CT imaging system having a tilting gantry mechanism to avoid directing x-ray radiation toward a subject's eyes. Other examples include where a head holder includes an adjustable mechanism having a lock to maintain a selected angle. The multi-positioner holder of the present disclosure ensures optimal positioning for neuro imaging in a CT imaging system and it adds benefits in cases where the gantry is fixed and unable to tilt. This may be achieved by inserting one or more of the plurality of pads into the cradle of the multi-positioner holder. The multi-positioner holder may tilt the patient's head forward to align the brain anatomy with the scanners field of view. This minimizes radiation dose exposure to the eyes and may reduce image artifacts from dental implants. The insert may be exchanged on a per patient basis to achieve a desired vertical tilt of the imaging subject. The cradle, or frame, is relatively large and therefore allows for additional pads to support additional appendages such as wrists, hands, ankles, feet, and so on. As well, multi-positioner holder allows for additional pads to support infants or smaller animals, with the aforementioned benefits. In some examples, the pads may be color coded for providing the user with visual recognition allowing the user to select the appropriate pad(s) quickly. In some examples, using angled pads for positioning may be preferable to existing head holder designs as they provide a range of vertical tilt, stability, and reduced risk of pinching. In one example, selectable angles may include 0°, 15°, 30° and 45°, however, other ranges may be utilized without departing from the scope of the present disclosure.

The multi-positioner holder may be used during imaging scans to support and adjust the patient's head or extremity through a range of discrete angles. In one example, the multi-positioner holder may use a strap mechanism to capture one or more selected pads and fasten the captured pads to the holder, meaning that once the support assembly is properly positioned, the configuration is secured and the patient is not able to change the angle. Additionally, the multi-positioner holder may include a pair of slots on the opposing sidewalls. The opposing slots provide an opening through which an additional strap may be inserted for securing a position of the patient's head or extremity.

FIG. 3A through FIG. 10B show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

FIG. 1A illustrates an exemplary CT imaging system 100 configured for CT imaging. Particularly, the CT imaging system 100 is configured to image a subject 112 such as a patient, an inanimate object, one or more manufactured parts, and/or foreign objects such as dental implants, stents, and/or contrast agents present within the body. In one embodiment, the CT imaging system 100 includes a gantry 102, which in turn, may further include at least one x-ray source 104 configured to project a beam of x-ray radiation 106 (see FIG. 2) for use in imaging the subject 112 laying on a patient table or table 114. Specifically, the x-ray source 104 is configured to project the beam of the x-ray radiation 106 towards a detector array 108 positioned on the opposite side of the gantry 102. Although FIG. 1A depicts only a single x-ray source 104, in certain embodiments, multiple x-ray sources and detectors may be employed to project a plurality of beams of x-ray radiation 106 for acquiring projection data at different energy levels corresponding to the patient. In some embodiments, the x-ray source 104 may enable dual-energy gemstone spectral imaging (GSI) by rapid peak kilovoltage (kVp) switching. In some embodiments, the x-ray detector employed is a photon-counting detector which is capable of differentiating x-ray photons of different energies. In other embodiments, two sets of x-ray sources and detectors are used to generate dual-energy projections, with one set at low-kVp and the other at high-kVp. It should thus be appreciated that the methods described herein may be implemented with single energy acquisition techniques as well as dual energy acquisition techniques.

In certain embodiments, the CT imaging system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. As described further herein, in some examples the image processor unit 110 may use both an analytic image reconstruction approach such as FBP in addition to an iterative image reconstruction approach.

In some CT imaging system configurations, an x-ray source projects a cone-shaped x-ray radiation beam which is collimated to lie within an X-Y-Z plane of a Cartesian coordinate system and generally referred to as an "imaging plane." The x-ray radiation beam passes through an object being imaged, such as the patient or subject. The x-ray radiation beam, after being attenuated by the object, impinges upon an array of detector elements. The intensity of the attenuated x-ray radiation beam received at the detector array is dependent upon the attenuation of a radiation beam by the object. Each detector element of the array produces a separate electrical signal that is a measurement of the x-ray beam attenuation at the detector location. The attenuation measurements from all the detector elements are acquired separately to produce a transmission profile.

In some CT imaging systems, the x-ray source and the detector array are rotated with a gantry within the imaging plane and around the object to be imaged such that an angle at which the radiation beam intersects the object constantly changes. A group of x-ray radiation attenuation measurements, e.g., projection data, from the detector array at one gantry angle is referred to as a "view." A "scan" of the object includes a set of views made at different gantry angles, or view angles, during one revolution of the x-ray source and detector. It is contemplated that the benefits of the methods described herein accrue to medical imaging modalities other than CT, so as used herein the term "view" is not limited to the use as described above with respect to projection data from one gantry angle. The term "view" is used to mean one data acquisition whenever there are multiple data acquisitions from different angles, whether from a CT, positron emission tomography (PET), or single-photon emission CT (SPECT) acquisition, and/or any other modality including modalities yet to be developed as well as combinations thereof in fused embodiments.

The projection data is processed to reconstruct an image that corresponds to a two-dimensional slice taken through the object or, in some examples where the projection data includes multiple views or scans, a three-dimensional rendering of the object. One method for reconstructing an image from a set of projection data is referred to in the art as the filtered back projection technique. Transmission and emission tomography reconstruction techniques also include statistical iterative methods such as maximum likelihood expectation maximization (MLEM) and ordered-subsets expectation-reconstruction techniques as well as iterative reconstruction techniques. This process converts the attenuation measurements from a scan into integers called "CT numbers" or "Hounsfield units," which are used to control the brightness of a corresponding pixel on a display device.

To reduce the total scan time, a "helical" scan may be performed. To perform a "helical" scan, the patient is moved while the data for the prescribed number of slices is acquired. Such a system generates a single helix from a cone beam helical scan. The helix mapped out by the cone beam yields projection data from which images in each prescribed slice may be reconstructed.

As used herein, the phrase "reconstructing an image" is not intended to exclude embodiments of the present invention in which data representing an image is generated but a viewable image is not. Therefore, as used herein, the term "image" broadly refers to both viewable images and data representing a viewable image. However, many embodiments generate (or are configured to generate) at least one viewable image.

In one example, table 114 may comprise a multi-positioner holder 116 which may be combined with one or more of a plurality of pads to position and/or tilt an anatomy for imaging. In the example, the holder is shown supporting a head. In other examples, the holder may support an extremity. Previous examples of head holders may be bulky, complex, or subject to risk of pinching. Furthermore, the previous examples demand a greater amount of operator effort and the process may become cumbersome and time consuming. If a patient moves their head when positioned in the head holder of a previous example, the adjustment mechanism may release and a position of the head holder may change. This may result in the patient sliding off the table and into a CT gantry.

In one example of the present disclosure, as will be described below in greater detail with respect to FIG. 3A and subsequent figures, multi-positioner holder 116 is a head holder. The multi-positioner holder 116 may comprise a cradle including opposing sidewalls. In some examples, the cradle may be used to support a patient's head. In other examples, the cradle maybe used to support a patient's extremity. In some examples, the cradle may be used in combination with one or more of a plurality of pads shaped to seat against the cradle within a clearance formed between the opposing sidewalls. Using various combinations of pads may provide a range of tilts or angles for an imaging procedure.

Turning now to FIG. 1B, a perspective view of table 114 is shown. The table comprises a top surface 118 arranged opposite from a bottom surface 120. An opening 126 is arranged directly between the top surface 118 and the bottom surface 120. The opening 126 may be positioned below a portion of the table on which a patient's neck may rest such as neck portion 128. The table 114 further comprises a first angled surface 122 and a second angled surface 124. In this way, the table 114 may comprise a trapezoidal shape, however, it will be appreciated that the table 114 may comprise other shapes, such as rectangular, square, or the like without departing from the scope of the present disclosure. The opening 126 is configured to receive the multi-positioner holder. For example, the opening 126 may be referred to as a table mount for the multi-position holder. In some examples, the multi-positioner holder may be removably coupled with the table 114 via the opening 126.

FIG. 2 illustrates an exemplary imaging system 200 similar to the CT imaging system 100 of FIG. 1A. In accordance with aspects of the present disclosure, the imaging system 200 is configured for imaging a subject 204 (e.g., the subject 112 of FIG. 1A). In one embodiment, the imaging system 200 includes the detector array 108 (see FIG. 1A). The detector array 108 further includes a plurality of detector elements 202 that together sense the beam of x-ray radiation 106 (see FIG. 2) that pass through the subject 204 (such as a patient) to acquire corresponding projection data. Accordingly, in one embodiment, the detector array 108 is fabricated in a multi-slice configuration including the plurality of rows of cells or detector elements 202. In such a configuration, one or more additional rows of the detector elements 202 are arranged in a parallel configuration for acquiring the projection data.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the x-ray source 104 and the detector array 108 rotate, the detector array 108 collects data of the attenuated x-ray beams. The data collected by the detector array 108 undergoes pre-processing and calibration to condition the data to represent the line integrals of the attenuation coefficients of the scanned subject 204. The processed data are commonly called projections.

In some examples, the individual detectors or detector elements 202 of the detector array 108 may include photon-counting detectors which register the interactions of individual photons into one or more energy bins. It should be appreciated that the methods described herein may also be implemented with energy-integrating detectors.

The acquired sets of projection data may be used for basis material decomposition (BMD). During BMD, the measured projections are converted to a set of material-density projections. The material-density projections may be reconstructed to form a pair or a set of material-density map or image of each respective basis material, such as bone, soft tissue, and/or contrast agent maps. The density maps or images may be, in turn, associated to form a volume rendering of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the x-ray source 104. In certain embodiments, the control mechanism 208 further includes an x-ray controller 210 configured to provide power and timing signals to the x-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. In one example, the computing device 216 stores the data in a mass storage or storage device 218. The storage device 218, for example, may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the x-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates only one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a patient table, such as table 114, which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized x-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

Though a CT system is described by way of example, it should be understood that the present technology may also be used on other imaging modalities, such as x-ray imaging systems, magnetic resonance imaging (MRI) systems, nuclear medicine imaging systems, positron emission tomography (PET) imaging systems, single-photon emission computed tomography (SPECT) imaging systems, ultrasound imaging systems, and combinations thereof (e.g., multi-modality imaging systems, such as PET/CT or PET/MR imaging systems). The present discussion of a CT imaging modality is provided merely as an example of one suitable imaging modality.

The multi-positioner holder may be used with an imaging system having a table, such as illustrated with respect to FIGS. 1A-2. In some examples, the multi-positioner holder may be used in combination with one or more of a plurality of interchangeable pads to adjust a tilt angle of a head or extremity of a subject relative to a horizontal axis (e.g., see coordinate system 390 below).

Turning now to FIGS. 3A-3D, various views of an example of a multi-positioner holder 300 are shown. As described above, in one example, a patient, such as subject 112 illustrated in FIG. 1A, may have their head positioned on the multi-positioner holder 300 to maintain a desired tilt angle of their head for imaging. CT imaging system 100 merely represents one exemplary usage of the multi-positioner holder 300. As such, multi-positioner holder 300 may be a non-limiting example of the multi-positioner holder 116 of FIG. 1A.

A coordinate system 390 is shown comprising three-axes, namely an x-axis parallel to a horizontal direction, a y-axis parallel to a vertical direction, and a z-axis perpendicular to each of the x- and y-axes. For reference, the coordinate system 390 is included in FIGS. 3A-6B and FIGS. 8-10B. The multi-positioner holder 300 comprises a central axis 399, which lies in an x-z plane.

The multi-positioner holder 300 comprises a head cradle or cradle 302 which may receive a head of a patient. The cradle 302 comprises a base 304 with a first sidewall, or first side 306, and a second sidewall, or second side 308, extending therefrom. An opening 396 is formed between the first side 306 and the second side 308. The base 304 comprises a substantially planar surface, or planar surface 328, against which the patient's head may rest within the opening 396. In one example, the cradle 302 may receive an extremity of a patient. The first side 306 and opposing, second side 308 may extend from opposite edges of the base 304, wherein the sides are curved at the edges and flatten as they extend upward. The first side 306 and the second side 308 may function as boundaries that block the patient from moving their head off of the multi-positioner holder 300. The multi-positioner holder 300 includes a first end 392 and a second end 394. When positioned in a CT system, such as CT imaging system 100 in FIG. 1A, the first end 392 faces the table and the second end 394 faces the gantry. The cradle 302 includes a front face curve 336 and a rear curve 338. The multi-positioner holder 300 includes an outer surface 330 of the cradle 302. In one example, the outer surface 330 is an outward facing surface, e.g., an exterior, of the first side 306, the base 304, and the second side 308.

In one example, a table attachment 310 may be continuous with the base 304 on the first end 392 of the cradle 302. In one example, the table attachment 310 may be inserted into a table mount, such as the opening 126 in FIG. 1B, or other receiving component of the table. A first extension 322 of the table attachment 310 may extend angularly upward toward the cradle 302. In one example, the table attachment 310 is physically coupled to a portion of the table on which a patient's neck may rest such as neck portion 128 in FIG. 1B. In one example, the cradle 302, the first extension 322, and the table attachment 310 are formed from carbon fiber. In one example, the cradle 302, the first extension 322, and the table attachment 310 are inflexible (e.g., rigid) due to the carbon fiber. In one example, the cradle 302 may include a side material 326 affixed to the outer surface 330. A table mount lock and release mechanism or mount lock 332 may fasten the table attachment 310 to the table when inserted into the table mount. For example, the mount lock 332 may apply pressure to the bottom surface of the table mount, such as the bottom surface 120 of the opening 126 in FIG. 1B. In one example, the mount lock 332, which is physically coupled to the table attachment 310, comprises a different material or materials compared to the cradle 302, such as plastic, metal, rubber, and combinations thereof.

The first side 306 may include a first pair of slots 312, shown as a first slot 316a positioned above a second slot 318a. The second side 308 may include a second pair of slots 314 oriented directly opposite the central axis 399 from the first pair of slots 312, shown as first slot 316b positioned above a second slot 318b. In one example, a strap 320 may be inserted through the first slots 316a, 316b for securing a patient's head or extremity when positioned at a first angle. In another example, the strap 320 may be inserted through the second slots 318a, 318b for securing a patient's head or extremity when positioned at a second angle. The strap 320 may include a strap end 324 that attaches to (e.g., fastens to) the side material 326 of the outer surface 330. For example, the side material 326 of the cradle 302 may be a first material and the strap end 324 of the strap 320 may be a second material, where the first material mates with the second material. As one example, the strap end 324 of the strap 320 and the side material 326 of the cradle 302 may be a hook and loop material, such as Velcro or similar.

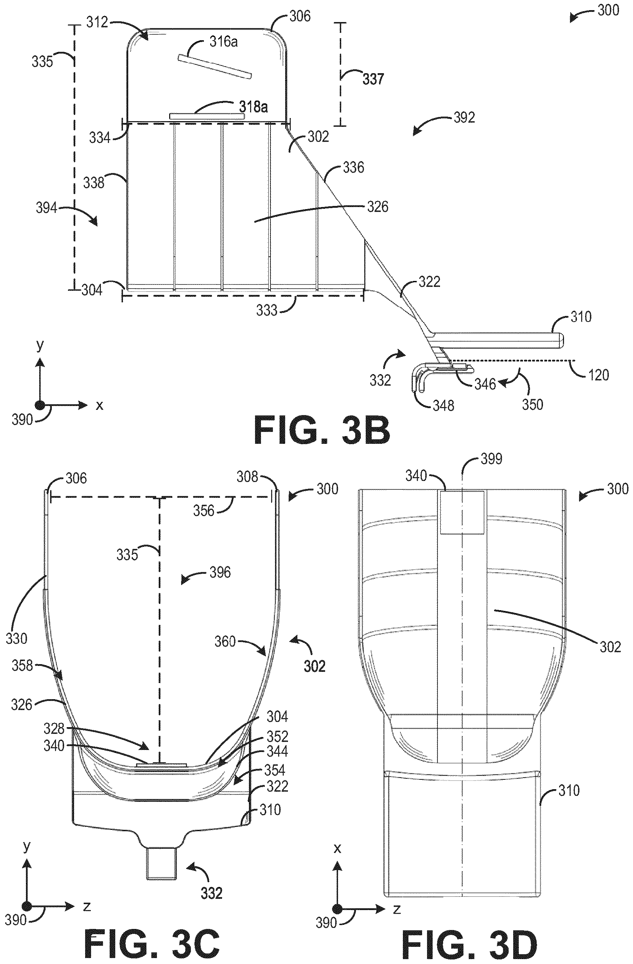

FIG. 3B shows a side profile view of the multi-positioner holder 300 looking down the z-axis. In some examples, dimensions of the first side 306, and mirroring second side 308, include a first dimension 333 that is greater than a second dimension 334. In other words, in profile, the first side 306 of the cradle 302 is wider nearer the base 304, narrowing as the side extends upward along the y-axis. In one example, the first pair of slots 312 are positioned in the narrower, upper portion of the first side 306 defined by dimension 337 and second dimension 334. The second pair of slots 314 are positioned similarly on the second side 308 (in FIG. 3A). A third dimension 335 of the first side 306, e.g., along the y-axis, may be slightly longer than the first dimension 333. In profile, the rear curve 338 on the second end 394 of the cradle 302 is perpendicular with respect to the planar surface 328 of the base 304. Opposite from the rear curve 338, the front face curve 336 is angled with respect to the planar surface 328 (in FIG. 3A) of the base 304. For example, the front face curve 336 is approximately -45 degrees from perpendicular with respect to the planar surface 328 of the base 304. In other examples, the front face curve 336 may be shaped differently.

In some examples, the mount lock 332 comprises a protrusion 346 pressed against the bottom surface 120 of the table (e.g., see FIG. 1B). A force of the protrusion 346 may block inadvertent dismounting of the multi-positioner holder 300 from the table. That is to say, the multi-positioner holder 300 may be fixedly mounted to the table until a tab 348 is actuated, resulting in actuation of the protrusion 346 in a direction away from the bottom surface 120, as illustrated via arrow 350. When the protrusion 346 is not pressed against the bottom surface 120, the multi-positioner holder 300 may be dismounted (e.g., removed) from the table. As such, the mount lock 332 may allow quick release of the multi-positioner holder 300 from the table such that a different holder may be mounted to for a subsequent patient.

FIG. 3C shows a third view of the multi-positioner holder 300 looking down the x-axis. From the third view, the first side 306 and second side 308 are curved and the base 304 is substantially planar. Looking down the x-axis, the cradle 302 is relatively narrow. For example, a height of the cradle 302, e.g., third dimension 335, is greater than a width 356. In some examples, the multi-positioner holder 300 may be used with one or more of plurality of pads, examples of which will be illustrated and discussed below. In some examples, an outer surface of the plurality of pads conforms to an inner surface of the cradle 302 such that the pads fit snugly inside the opening 396 formed by the first side 306, second side 308, and base 304 of the multi-positioner holder 300. In one example, the inner surface of the cradle 302 may include the planar surface 328, a first inner surface 358 comprising the interior surface of the first side 306, and a second inner surface 360 comprising the interior surface of the second side 308. A fastening material or first fastener 340 may be positioned on, e.g., affixed to, the planar surface 328 of the base 304. In one example, the first fastener 340 may be similar to the first material forming the side material 326 of the outer surface 330 of the cradle 302. In another example, the first fastener 340 may be similar to the material forming the strap end 324 of the strap 320.

Sloped portion 344 of the cradle 302 connects the base 304 with the first extension 322. The sloped portion 344 curves similarly to the cradle 302, however the sloped portion 344 is at angled with respect to the base 304 and the table attachment 310. The surface 354 of the first extension 322 may make face sharing contact with a mounting surface of the table (e.g., surfaces surrounding opening 126 in FIG. 1B), whereas the angled surface 352 of the sloped portion 344 may be exposed and provide a resting surface for the patient's neck or extremity.

FIG. 3D illustrates fourth view of the multi-positioner holder 300 looking down the y-axis. From the fourth view, the first fastener 340 is positioned adjacent to the second end 394 of the cradle 302. In one example, the first fastener 340 may be a hook and loop material or similar. The pads may have a mating fastener, also formed of hook and loop material or similar. For example, the first fastener 340 of the multi-positioner holder may attach to a second fastener of a pad (e.g., see FIGS. 4B, 5B, 6B, etc.). Additionally or alternatively, the first fastener 340 may mate with a strap for securing the pads to the multi-positioner holder 300 (e.g., see strap 914 in FIG. 9A).

FIG. 4A through FIG. 6B illustrate first, second, and third examples, respectively, of a stacking pad. In one example, the stacking pads or pads may be used singly or in combination, e.g., stacked, one on top of another, to support a head or extremity of a patient. FIG. 4A and FIG. 4B show an example of a first pad, a patient interface head pad 400 for positioning a patient's head at a first angle. FIG. 5A and FIG. 5B show an example of a patient interface extremity pad 500 for positioning a patient's extremity. In one example, the patient interface head pad 400 and the patient interface extremity pad 500 may position a patient body part at an approximately 0° angle. FIG. 6A and FIG. 6B show an example an angled positioning pad 600 for increasing a tilt angle of a patient head or extremity. In one example, the angled positioning pad may be a second pad for positioning the patient's head or extremity at a second angle, different from the first angle. In one example, the second angle may be greater than 0°, such as 5°, 10°, or 15°. In another example, the patient interface head pad 400, the patient interface extremity pad 500, and the angled positioning pad 600 may be color coded for ease of use. For example, the patient interface head pad 400 may be a first color such as green. The patient interface extremity pad 500 may be a second color, different from the first color, such as blue. The angled positioning pad 600 may be yet a different color such as red. Color coding may have advantage of enabling the user, such as a physician or a technician, to quickly select an appropriate stacking pad. In one example, the stacking pads may be formed from a foam material including rubber, polyethylene, polyurethane, or polystyrene based foams. Stacking pads formed from other materials have been imagined.

FIG. 4A illustrates a front perspective view of the patient interface head pad 400. In one example, the patient interface head pad 400 may be positioned in a head holder of an imaging device, such as the multi-positioner holder 116 for CT imaging system 100 illustrated with respect to FIG. 1A. As another example, the pad may be positioned in the opening 396 of the cradle 302 of the multi-positioner holder 300 illustrated with respect to FIGS. 3A-3D. In yet other examples, patient interface head pad 400 may be positioned directly on a patient table. In one example, the patient interface head pad 400 provides a 0° angle for a patient head to rest during a procedure. The patient interface head pad 400 comprises a central axis 499, which lies in an x-z plane.

The patient interface head pad 400 comprises a first pad cradle, or cradle 402, which may receive a head of a patient. The cradle 402 comprises a first body, or body 404, having first opposing sidewalls extending therefrom. The first opposing sidewalls comprise a first side 406 and a second side 408. A pad opening 434 is formed between the first side 406 and the second side 408. The body 404 comprises a substantially planar surface, or planar surface 410, against which the patient's head may rest within the pad opening 434. The first side 406 and the second side 408 may extend from opposite edges of the body 404. The patient interface head pad 400 includes a first end 412 and a second end 414. When positioned in a CT system, such as shaped to seat against the cradle in FIG. 1A, the first end 412 faces the table and the second end 414 faces the gantry.

In one example, dimensions of the patient interface head pad 400 include a first dimension 422 that is greater than a second dimension 424. In other words, the sides 406, 408 of the cradle 402 include a laterally wider, first region 428 nearer the body 404 and a laterally narrower, second region 430 as the sides extends upward along the y-axis. The wider, first region 428 forms a curve joining the second region 430 of the sides 406, 408 and the body 404. A third dimension 426 of the cradle 402, e.g., a cradle height, may be less than the first dimension 422, e.g., a cradle length. In one example, the opposing sidewalls and the body 404 may have a similar thickness throughout. For example, a material thickness 432 of the first side 406, the second side 408, and the body 404 may be approximately the same throughout the patient interface head pad 400. The patient interface head pad 400 includes a width 435 of the body 404. In one example, the width 435 of the body 404 may be similar to the second dimension 424 of the sides 406, 408.

The patient interface head pad 400 includes an outer surface 420. In one example, the outer surface 420 is an outward facing, e.g., exterior, surface of the first side 406, the body 404, and the second side 408. Positioned in the multi-positioner, the outer surface 420 makes face sharing contact with the interior surface of the cradle 302. An interior surface of the pad comprises the planar surface 410 and side surface 418. A perimeter surface 438 joins the interior surface and the outer surface 420. In one example, the perimeter surface 438 may meet the outer surface 420 at an acute angle. In some use cases, the perimeter surface 438 along the second end 414 may be approximately flush with the rear curve 338 of the multi-positioner holder 300.

FIG. 4B illustrates an underside view 450 of the patient interface head pad 400. Particularly, the underside view 450 shows the patient interface head pad 400 viewed from the second end 414. The underside view 450 shows the side surface 418, the outer surface 420, and the perimeter surface 438 therebetween. Looking down the x-axis, the pad opening 434 may be u-shaped.

A recess 452 formed in the in the body underside 456 may provide a depression where a fastening material may be placed. In one example the fastening material may be a second fastener 454. In one example, the recess 452 may be positioned adjacent to the second end 414 of the cradle 402. In one example, the second fastener 454 may be a mating material that interacts with a fastening material positioned in the cradle interior of the multi-positioner holder (e.g., see FIG. 3C-3D) or positioned in the cradle interior of an angled positioning pad (e.g., see FIG. 6A and FIG. 6B). For example, the second fastener 454 may make face sharing contact with the first fastener 340 for securing the patient interface head pad to the multi-positioner holder 300. In one example, the second fastener 454 may be made of a hook and loop material or similar.

FIG. 5A illustrates a front perspective view of the patient interface extremity pad 500. In one example, the patient interface extremity pad 500 may be positioned in a head holder of an imaging device, such as the multi-positioner holder 116 for CT imaging system 100 illustrated with respect to FIG. 1A. As another example, the patient interface extremity pad 500 may be positioned in the cradle 302 of the multi-positioner holder 300 illustrated with respect to FIGS. 3A-3D. In yet other examples, patient interface extremity pad 500 may be positioned directly on a patient table. In one example, the patient interface extremity pad 500 provides a 0° angle for a patient extremity to rest during a procedure. The patient interface extremity pad 500 comprises a central axis 599, which lies in an x-z plane.

The patient interface extremity pad 500 comprises an extremity pad cradle or cradle 502, which may receive a patient extremity. The cradle 502 includes a body 504 with a first sidewall, or first side 506, and a second sidewall, or second side 508, extending therefrom. A pad opening 534 is formed between the first side 506 and the second side 508. The body 504 comprises a substantially planar surface or planar surface 510 against which the patient's extremity may rest within the pad opening 534. The first side 506 and opposing, second side 508 may extend from opposite edges of the body 504. The patient interface extremity pad 500 includes a first end 512 and a second end 514. When positioned in a CT system, such seated against the cradle in FIG. 1A, the patient interface extremity pad 500 may be configured in a first position or a second position. For example, when configured in the first position, the first end 512 may face the gantry and when configured in the second position the first end 512 may face the table.

In one example, looking down the z-axis, the sides 506, 508 of the patient interface extremity pad are approximately trapezoidal. For example, the second side 508, and is formed with a first base 542 of a first dimension 522 that is approximately parallel with second base 544 of a second dimension 524. The first dimension 522 is more than twice magnitude of the second dimension 524. The second side 508 includes a first side 546 of a third dimension 526. The first side 546 is arranged approximately perpendicular to the first base 542 and the second base 544. The second side 508 of the patient interface extremity pad 500 includes a second side 548 of a fourth dimension 550. The second side 548 is arranged at an acute angle with respect to the first base 542 and an obtuse angle with respect to the second base 544. The sides 506, 508 of the cradle 502 include a first region 528 nearer to the body 504 and a second region 530 further from the body 504 along the y-axis. In one example, a width 532 of the body 504 may be greater than the second dimension 524 and less than the first dimension 522 of the sides 506, 508.

The patient interface extremity pad 500 includes the planar surface 510 of the body 504, a second interior surface 518 in the first region 528 of the sides 506, 508, a third interior surface 552 in the second region 530 of the sides 506, 508, and an outer surface 520. In one example, the outer surface 520 is an outward facing, e.g., exterior, surface of the first side 506, the body 504, and the second side 508. In one example, positioned in the multi-positioner, the outer surface 520 makes face sharing contact with the interior surface of the cradle. A perimeter surface 538 joins the interior surfaces (e.g., the planar surface 510, the second interior surface 518, and the third interior surface 552) and the outer surface 520.

The perimeter surface 538 comprises a ridge 554, first angled surface 556, and second angled surface 558. In some examples, the perimeter surface 538 along the first end 512 may be approximately flush with the front face curve 336 of the multi-positioner holder 300. In some examples, the perimeter surface 538 along the second end 514 may be approximately flush with the rear curve 338 of the multi-positioner holder 300.

FIG. 5B illustrates an underside view 560 of the patient interface extremity pad 500. The underside view 560 shows the patient interface extremity pad 500 as seen from the second end 514. The underside view 560 shows the second interior surface 518, the third interior surface 552, the outer surface 520, and the perimeter surface 538 therebetween.

In some aspects, the cradle 502 and the body 504 of the patient interface extremity pad 500 may be similar to the cradle 402 and the body 404 of the patient interface head pad 400. For example, the outer surfaces (e.g., outer surface 420, outer surface 520) have a similar shape and the body 404, 504 is horizontal relative to the cradle sidewalls. In one example, the patient interface extremity pad 500 is formed with at least a portion of the sidewalls having an irregular thickness. For example, the first side 506 and the second side 508 may be formed having a first thickness 562 in the first region 528 of the pad, increasing to a second, greater thickness 566 in the second region 530. In other examples, the patient interface extremity pad may be formed with a wall having a material thickness that is approximately the same, e.g., similar, throughout.

The cradle 502 has a u-shaped, outer edge 570 and an irregular, inner edge 572. The inner edge 572 is narrower near the body 504, wider towards an upper portion 568 of the sides 506, 508, and narrowest at a transition 584 between the first region 528 and the second region 530 of the sides 506, 508. Correspondingly, the pad opening 534 may be approximately the width 532 near the body 504, narrowing to a second width 574 at the transition 584, and increasing to third width 576 towards the upper portion 568 of the sides 506, 508.

A recess 578 formed in the in the body underside 580 may provide a depression where a fastening material may be placed. In one example, the fastening material may be a second fastener 582. The recess 578 may be positioned adjacent to the second end 514 of the cradle 502. In one example, the second fastener 582 may be made from the same or similar material as the second fastener 454, and similarly, may mate with a fastener positioned in the cradle interior of the multi-positioner holder and/or may mate with a fastener positioned on an angled positioning pad (e.g., see FIG. 6A and FIG. 6B below). For example, the second fastener 582 may make face sharing contact with first fastener 340 for securing the patient interface extremity pad 500 to the multi-positioner holder 300.

FIG. 6A illustrates a front perspective view of the angled positioning pad 600. In one example, the angled positioning pad 600 may be positioned in a head holder of an imaging device, such as the multi-positioner holder 116 for CT imaging system 100 illustrated with respect to FIG. 1A. As another example, the angled positioning pad 600 may be positioned in the cradle 302 of the multi-positioner holder 300 illustrated with respect to FIGS. 3A-3D. In yet other examples, the angled positioning pad 600 may be positioned directly on a patient table. In one example, the angled positioning pad 600 provides a greater than 0° tilt angle 652 (in FIG. 6B) for a patient head or extremity to rest during a procedure. The angled positioning pad 600 comprises a central axis 699, which lies in an x-z plane.

The angled positioning pad 600 comprises a second pad cradle, or cradle 602, which may receive a patient head or extremity. Additionally or alternatively, the angled positioning pad 600 may receive one or more of a patient interface head pad, a patient interface extremity pad, or a second angled positioning pad, such as shown in more detail in FIGS. 8A-10B. The cradle 602 comprises a second body, or body 604, having second opposing sidewalls extending therefrom. The second opposing sidewalls comprise a first side 606 and a second side 608. The first side 606 and opposing, second side 608 may extend from opposite edges of the body 604. A pad opening 636 is formed between the first side 506 and the second side 508. The body 604 comprises an interior surface 610 against which the patient or the one or more additional pads may rest within the pad opening 636. The body 604 further comprises an outer surface 620. In one example, the outer surface 620 is an outward facing surface of the first side 606, the body 604, and the second side 608. A perimeter surface 638 joins the interior surface 610 and the outer surface 620.

The angled positioning pad 600 includes a first end 612 and a second end 614. When positioned in a CT system, such seated against the cradle in FIG. 1A, the angled positioning pad 600 may be configured in a first position or a second position. For example, when configured in the first position, the first end 612 may face the gantry and when configured in the second position the first end 612 may face the table. In some use cases, along the first end 612, the perimeter surface 638 may be approximately flush with the front face curve 336 of the multi-positioner holder 300. Facing the second end 614 of the angled positioning pad 600, the perimeter surface 638may be approximately flush with the rear curve 338 of the multi-positioner holder 300.

The angled positioning pad 600 is formed having variable material thickness throughout. For example, the thickness of the body 604 of the pad may increase from the first end 612 towards the second end 614. The thickness of the sides 606, 608 may increase from an upper portion 646 of the cradle 602 towards the body 604.

A first recess 640 is formed in the in body 604 and may provide a depression where a fastening material may be placed. In one example, the fastening material may be a third fastener 642. In one example, the first recess 640 may be positioned adjacent to the second end 614 of the cradle 602. In one example, the third fastener 642 may mate with material positioned similarly on the outer surfaces of the patient interface head pad 400 and the patient interface extremity pad 500 (e.g., see FIG. 4B and FIG. 5B). For example, the third fastener 642 may make face sharing contact with second fastener 454 for securing the patient interface head pad 400 to angled positioning pad 600. The third fastener 642 may be made of hook and loop material or similar.

FIG. 6B illustrates cross section 650 of the angled positioning pad 600. Particularly, the cross section 650 shows an example tilt angle 652 of the body 604 and example fasteners. The cross section 650 shows the increasing thickness, e.g., at tilt angle 652, of the body 604 from the first end 612 to the second end 614. The tilt angle 652 may provide a greater than 0° angle for a patient head or extremity to rest during a procedure. In one example, the tilt angle may be 15°.

In one example, looking down the z-axis, the angled positioning pad 600 is approximately trapezoidal. For example, the second side 608 is formed with the first base 654 of a first dimension 662 that is approximately parallel with a second base 656 of a second dimension 664. The first dimension 662 is approximately twice magnitude of the second dimension 664. The second side 608 includes a first side 658 of a third dimension 666. The first side 658 is arranged approximately perpendicular to the first base 654 and the second base 656. The second side 608 of the angled positioning pad 600 includes a second side 660 of fourth dimension 668. The second side 660 is arranged at an acute angle with respect to the first base 654 and an obtuse angle with respect to the second base 656. The second side 608 further includes an upper extension 670 having a height of a fifth dimension 672 and a length of the second dimension 664. The upper extension 670 may be continuous with a lower portion 674 of the second side 608. The first side 606 includes a matching, upper extension (e.g., 607 in FIG. 6A). In one example, the upper extensions are rectangular shaped and may provide additional support. In some examples, the upper extensions may be shaped.

The cross section 650 shows the depression formed by first recess 640 and the third fastener 642 placed therein. Similarly, a second recess 676 may form a depression in the first base 654. An additional fastener 643 may be positioned in the second recess 676. In one example, the first recess 640 and the second recess 676 may be positioned adjacent to the second end 614 of the pad. In one example, the third fastener 642 may mate with and secure to the second fastener 454 positioned on the lower surface of the patient interface head pad 400 and the second fastener 582 positioned on the patient interface extremity pad 500. The additional fastener 643 may be the same or similar to the second fastener 454 and the second fastener 582. For example, the additional fastener 643 may mate with the first fastener 340 positioned in the cradle of the multi-positioner holder (e.g., see FIG. 3C and FIG. 3D).

In one example, the fastening materials may comprise fasteners and receivers. As one example, the first fastener 340 of the multi-positioner holder 300 may be a first fastening material where the first fastening material is a fastener. The additional fastener 643 of the angled positioning pad 600 may be a second fastening material where the second fastening material is a receiver. For example, the receiver is a contrasting material relative to the fastener that mates with the fastener. In such an example, the additional fastener 643 making contact with the first fastener 340 couples (e.g., attaches, fastens) the angled positioning pad 600 to the multi-positioner holder 300. Similarly, as another example, the third fastener 642 of angled positioning pad 600 may the first fastening material, e.g., another fastener. The second fastener 454 of the patient interface head pad 400 may be the second fastening material, e.g., another receiver. In such an example, the second fastener 454 making contact with the third fastener 642 couples to the patient interface head pad 400 to the angled positioning pad 600.

Turning now to FIGS. 7A, 7B, and 7C, a first position 700, a second position 725, and a third position 750, respectively, of a multi-positioner holder for a patient table used in combination with one or more stacking pads are shown. In one example, the patient table may be the same or similar to the table 114 illustrated with respect to FIGS. 1A-1B. The multi-positioner holder may be the same or similar to the multi-positioner holder 300 illustrated with respect to FIGS. 3A-3D. The stacking pads may be the same or similar to the patient interface head pad 400 illustrated with respect to FIGS. 4A-4B and the angled positioning pad 600 illustrated with respect to FIGS. 6A-6B. In one example, the positions are achieved by using the patient interface head pad 400 singly and in combination with, e.g., by stacking, one or more of the angled positioning pad 600.

Turning now to FIG. 7A, in the first position 700, a multi-positioner holder 702 may comprise an angle 712 of 0° degrees. As such, the multi-positioner holder 702 may be in line with a table 704 to which a table attachment 706 is physically coupled. By doing this, a patient's head 710 may be relatively in line with their spine. In the first position 700, the multi-positioner holder 702 is used in combination with the patient interface head pad 708.

Turning now to FIG. 7B, in a second position 725, the multi-positioner holder 702 may comprise an angle 730, which is greater than zero. In one example, the angle 730 is between 5 and 30 degrees. In some examples, the angle 730 is between 10 and 20 degrees. In one example, the angle 730 is equal to exactly 15 degrees. In the second position 725, the multi-positioner holder 702 is used in combination with the patient interface head pad 708 and a first angled positioning pad 732.

The multi-positioner holder 702 may be adjusted to the second position 725, from the first position 700, via inserting the first angled positioning pad 732 between the patient interface head pad 708 and the cradle interior (e.g., cradle 302 in FIG. 3A) of the multi-positioner holder 300. By doing this, a tilt of the patient's head 710 may increase relative to the first position 700. This may be desired during imaging to avoid various artifacts that may arise due to dental work and the like.

FIG. 7C shows, in the third position 750, the multi-positioner holder 702 may comprise an angle 755, which is greater than zero and greater than the angle 730. In one example, the angle 755 is between 10 and 60 degrees. In some examples, additionally or alternatively, the angle 730 is between 20 and 50 degrees. In one example, the angle 755 is 30 degrees. In the third position 750, the multi-positioner holder 702 is used in combination with the patient interface head pad 708, the first angled positioning pad 732 and a second angled positioning pad 752. The second angled positioning pad 752 may be the same or similar to the first angled positioning pad 732.

The multi-positioner holder 702 may be adjusted to the third position 750, from the first position 700 or the second position 725, via inserting the second angled positioning pad 752 beneath the patient interface head pad 708. For example, the second angled positioning pad 752 may be inserted below the patient interface head pad 708 and above the first angled positioning pad 732. As another example, the second angled positioning pad 752 may be inserted between the first angled positioning pad 732 and the cradle interior of the multi-positioner holder 702. By doing this, the tilt of the patient's head 710 may increase relative to each of the first position 700 and the second position 725. As one example, one of the angle 712, the angle 730 and the angle 755 may be selected based on an imaging of the patient's head 710 that captures the fewest artifacts.

In one example, the stacking pads may be secured in place by mating fasteners, such as the mating fasteners shown in FIGS. 3C-D, FIG. 4B, FIG. 5B, and FIG. 6B. A strap may be used to further secure the assembly, such as shown in FIG. 3A and FIG. 9A below.

FIG. 8 through FIG. 10B show example configurations for a multi-position holder used in combination with one or more stacking pads. The example configurations may be used with an imaging system having a patient table, such as the CT imaging system 100 including table 114 illustrated with respect to FIGS. 1A-2. The multi-positioner holder may be the same or similar to the multi-positioner holder 300 illustrated with respect to FIGS. 3A-3D. The stacking pads may be the same or similar to one or more of the patient interface head pad 400 illustrated with respect to FIGS. 4A-4B, the patient interface extremity pad 500 illustrated with respect to FIGS. 5A-5B, and the angled positioning pad 600 illustrated with respect to FIGS. 6A-6B. Components of the example configurations that are identical to components of the multi-positioner holder 300, the patient interface head pad 400, the patient interface extremity pad 500, and the angled positioning pad 600 are numbered the same and will not be introduced. As described above, in one example, a patient, such as subject 112 of FIG. 1A, may have a body part positioned on the multi-positioner holder 300 to maintain a desired tilt angle of the body part for imaging. In the example configurations, the tilt angle is achieved by using the patient interface head pad 400 or the patient interface extremity pad 500 in combination with, e.g., by stacking, one or more of the angled positioning pad 600.

Turning to FIG. 8, a first example configuration 800 includes the multi-positioner holder 300, the angled positioning pad 600, and the patient interface head pad 400. The example configuration 800 comprises a first end 802, a second end 804, and a central axis 899, which lies in an x-z plane.

The angled positioning pad 600 is positioned in the cradle 302. The patient interface head pad 400 is positioned over the angled positioning pad 600. The first end 412 of the patient interface head pad 400 and the first end 612 of the angled positioning pad 600 are aligned with the first end 392 of the multi-positioner holder 300. The second end 414 of the patient interface head pad 400 and the second end 614 of the angled positioning pad 600 are aligned with the second end 394 of the multi-positioner holder 300. Configured in this way, a positive tilt angle with respect to the table may be provided by the stacking pads. For example, the planar surface 410 of the patient interface head pad 400 is oriented at a tilt angle that is the sum of the tilt angle of the patient interface head pad 400 and the tilt angle of the angled positioning pad 600. In one example, the tilt angle illustrated in the example configuration 800 may be greater than 0° and less than 30° such as 15°. Other tilting angles have been imagined.

In one example, the mount lock 332 may secure the multi-positioner holder 300 to the table. With the multi-positioner holder 300 mounted, the patient may recline on the table to rest their head on the example configuration 800 from the first end 802. The second end 804 faces the imaging device. The planar surface 410 may support the head of the patient at the tilt angle that is the sum of the tilting angles of the stacking pads included in the example configuration 800. The planar surface 410, the perimeter surface 438, and angled surface 352 provide a resting surface for the patient's neck.

The head of the patient may be secured in place for imaging with the use of the strap 320. The strap 320 may be inserted through the first slots 316a, 316b for securing the patient's head when positioned at the tilt angle provided in the first example configuration 800. In one example, the tilt of the first slots 316a, 316b may be the same or similar to the tilt angle of the combination of the angled positioning pad 600 and the patient interface head pad 400, e.g., 15°. In one example, the strap 320 may secure to the multi-positioner holder 300 by attaching to the side material 326 of the outer surface 330.

Turning to FIG. 9A and 9B, a second example configuration 900 includes the multi-positioner holder 300, the first angled positioning pad 600, a second angled positioning pad 906, and the patient interface head pad 400. In one example, the second angled positioning pad 906 may be the same or similar to the first angled positioning pad 600. For example, the second angled positioning pad 906 may provide the same tilt angle as the first angled positioning pad 600. In other examples, the second angled positioning pad 906 may provide a second tilt angle, different from the first tilt angle. The second example configuration 900 comprises a first end 902 and a second end 904. A plane 916 indicates a longitudinal plane cross-section of the second example configuration 900. The cross-section is shown in FIG. 9B to illustrate the tilt angle of the second example configuration 900.

The first angled positioning pad 600 is positioned in the cradle 302 and the second angled positioning pad 906 is positioned over the first angled positioning pad 600. The patient interface head pad 400 is positioned over the second angled positioning pad 906. In one example, fastener connection 908 secures the first angled positioning pad 600 to the multi-positioner holder 300. In one example, the fastener connection 908 may comprise the first fastener 340 (e.g., in FIG. 3C-D) in face-sharing contact with the third fastener 642 (e.g., in FIG. 6A-6B). Fastener connection 910 secures the first angled positioning pad 600 to the second angled positioning pad 906. In one example, the fastener connection 910 may comprise the additional fastener 643 in face-sharing contact with (a second) of the third fastener 642 (e.g., in FIG. 6A-6B). Fastener connection 912 secures the second angled positioning pad 906 to the patient interface head pad 400. In one example, the fastener connection 912 may comprise the third fastener 642 in face-sharing contact with second fastener 454 (e.g., in FIG. 4B). Additionally or alternatively, a strap 914 may secure the plurality of stacking pads to the multi-positioner holder 300 in the second example configuration 900. For example, the strap 914 may include at least a portion formed from a fastening material, such as hook and loop, that mates with the fastener connection 912 and the side material 326 of the outer surface 330.

FIG. 9B is a cross-section view 950 of the second example configuration 900. The cross-section view 950 is shown looking into the z-axis to illustrate an exemplary second tilt angle 952 provided by the combined stacking pads positioned in the multi-positioner holder 300. Similar to the first example configuration 800, the stacking pads are oriented to provide a positive tilt angle with respect to the table. For example, the first end 412 of the patient interface head pad 400 is aligned with the first end of the multi-positioner holder 300, and the first ends of the stacking pads captured therebetween. The planar surface 410 of the patient interface head pad 400 is oriented at the second tilt angle 952 that is the sum of the tilt angle of the patient interface head pad 400, the tilt angle of the angled positioning pad 600, and the tilt angle of the second angled positioning pad 906. In one example, the second tilt angle 952 may be greater than 0° and less than 45° such as 30°. Other tilt angles have been imagined.

FIG. 10A and 10B illustrate a third example configuration 1000. Contrasting with FIGS. 8-9B, where a first orientation of one or more angled positioning pads for producing a positive tilt angle is shown, the third example configuration 1000 shows a second orientation of the angled positioning pads for producing a negative tilt angle. The third example configuration 1000 comprises the multi-positioner holder 300, the first angled positioning pad 600, the second angled positioning pad 906, and the patient interface extremity pad 500. The third example configuration 1000 further comprises a first end 1002 and a second end 1004. A plane 1006 indicates a longitudinal plane cross-section of the third example configuration 1000. The cross-section is shown in FIG. 10B to illustrate the tilt angle of the third example configuration 1000.

Turning to FIG. 10A, the first angled positioning pad 600 is positioned in the cradle 302 and the second angled positioning pad 906 is positioned over the first angled positioning pad 600. The patient interface extremity pad 500 is positioned over the second angled positioning pad 906. When mounted to the table of the imaging device the first end 1002 of the third example configuration 1000 faces the table and the second end 1004 faces the gantry. However compared to the first example configuration 800 and the second example configuration 900, the stacking pads are reversed to provide a negative tilt angle. For example, the first end 512 of the patient interface extremity pad 500 and the first end 612 of the angled positioning pad 600 are aligned with the second end 394 of the multi-positioner holder 300. Similarly, the second end 514 of the patient interface extremity pad 500 and the second end 614 of the angled positioning pad 600 is aligned with the first end 392 of the multi-positioner holder 300.

FIG. 10B is a cross-section view 1050 of the third example configuration 1000. The cross-section view 1050 is shown looking into the z-axis to illustrate the exemplary negative tilt angle 1052 provided by the combined stacking pads positioned in the multi-positioner holder 300 in the reverse orientation. The planar surface 510 of the patient interface head pad 400 is oriented at the negative tilt angle 1052 that is the sum of the tilt angle of the patient interface extremity pad 500, the tilt angle of the angled positioning pad 600, and the tilt angle of the second angled positioning pad 906. A negative tilt angle may be desirable in some patient imaging scenarios.

In one aspect, a multi-positioner holder may position and support a patient head or extremity and provide a range of discrete angles when used in combination with one or more pads. The combination of the pads, color coding, and the holder may allow quick, precise, and optimal positioning of a patient head or extremity. The technical effect of using the multi-positioner holder and optional pads is to allow repeatable scans of a target area while allowing an operator to easily and stably adjust the tilt of the scanned subject with minimal patient disturbance and reduced incidence of pinching. By doing this, medical imaging may be enhanced and patient outcomes improved.

The disclosure also provides support for a system comprising: a support assembly for use with an imaging system, the support assembly comprising: a cradle including a base and opposing sidewalls, and a plurality of pads shaped to seat against the base within an opening formed between the opposing sidewalls, where each pad of the plurality of pads supports a head or extremity of a subject to be imaged at a different angle relative to the base. In a first example of the system, an outer surface of the plurality of pads conforms to an inner surface of the cradle. In a second example of the system, optionally including the first example, a width of the cradle is less than a length of the cradle and a height of the cradle is greater than the length of the cradle. In a third example of the system, optionally including one or both of the first and second examples, a first pad of the plurality of pads has a first color and is shaped to support the head or extremity at a first angle, and a second pad of the plurality of pads has a second color, different from the first color, and is shaped to support the head or extremity at a second angle, different from the first angle. In a fourth example of the system, optionally including one or more or each of the first through third examples, the first pad comprises a first pad cradle including first opposing sidewalls and a first body, and where the first body is horizontal relative to the base of the cradle. In a fifth example of the system, optionally including one or more or each of the first through fourth examples, the second pad comprises a second pad cradle including second opposing sidewalls and a second body, wherein the second opposing sidewalls increase in thickness towards the second body, and the second body increases in thickness from a first end to a second end. In a sixth example of the system, optionally including one or more or each of the first through fifth examples, the first opposing sidewalls and the first body have a similar thickness throughout. In a seventh example of the system, optionally including one or more or each of the first through sixth examples, at least a portion of the first opposing sidewalls have an irregular thickness. In an eighth example of the system, optionally including one or more or each of the first through seventh examples the opposing sidewalls further comprising a first sidewall and a second sidewall mirroring the first sidewall, wherein the first sidewall has a first slot positioned directly above a second slot, and wherein the first slot is oriented horizontal relative to the base of the cradle and the second slot is oriented at an angle relative to the first slot. In a ninth example of the system, optionally including one or more or each of the first through eighth examples, the angle of the second slot, and a tilt angle of a first pad of the plurality of pads are similar.

The disclosure also provides support for a support assembly for use with an imaging system, the support assembly comprising: a cradle including a base and opposing sidewalls, and a plurality of pads shaped to seat against the cradle within an opening formed between the opposing sidewalls, and where a range of discrete angles are achieved by stacking one or more pads of the plurality of pads in the cradle, where a first pad of the plurality of pads has a first angle relative to the base of the cradle, and where a second pad of the plurality of pads has a second angle relative to the base of the cradle. In a first example of the system, the plurality of pads are formed from a foam material. In a second example of the system, optionally including the first example, the cradle is formed from carbon fiber and an outer surface of the cradle has a fastening material affixed thereto. In a third example of the system, optionally including one or both of the first and second examples, a first fastener is positioned on an inner surface of the cradle, a second fastener is positioned on a lower surface of the first pad and the lower surface of the second pad, and a third fastener is positioned on the inner surface of the second pad. In a fourth example of the system, optionally including one or more or each of the first through third examples, face-sharing contact between the first fastener and the second fastener secures the first pad or the second pad to the cradle, and face-sharing contact between the second fastener and the third fastener secures the first pad to the second pad. In a fifth example of the system, optionally including one or more or each of the first through fourth examples, the imaging system is one of a computerized tomography (CT) imaging system, positron emission tomography (PET) imaging system, single-photon emission CT (SPECT) imaging system, or magnetic resonance imaging (MRI) system.

The disclosure also provides support for a system, comprises: a patient table, and a support assembly selectively coupled to the patient table, where the support assembly comprises: a cradle including a base and opposing sidewalls, and a plurality of pads shaped to seat against the cradle within an opening formed between the opposing sidewalls. In a first example of the system, a first orientation of a first pad of the plurality of pads relative to the patient table produces a positive tilt angle and a second orientation of the first pad relative to the patient table produces a negative tilt angle. In a second example of the system, optionally including the first example, a tilt angle of a head or extremity of a subject is adjusted relative to a horizontal axis. In a third example of the system, optionally including one or both of the first and second examples the cradle further comprising an angled surface, where the angled surface supports a neck or extremity of a subject.

As used herein, an element or step recited in the singular and proceeded with the word "a" or "an" should be understood as not excluding plural of said elements or steps, unless such exclusion is explicitly stated. Furthermore, references to "one embodiment" of the invention do not exclude the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A system comprising:
a support assembly for use with an imaging system (100), the support assembly comprising:
a cradle (302) including a base (304) and opposing sidewalls (306, 308); and
a plurality of pads (400, 500, 600) to seat against the base (304) within an opening (396) formed between the opposing sidewalls (306, 308), where each pad of the plurality of pads supports a head or extremity of a subject (112) to be imaged at a different angle relative to the base.

2. The system of claim 1, wherein an outer surface (420, 520, 620) of the plurality of pads (400, 500, 600) conforms to an inner surface (328, 358 360) of the cradle (302).

3. The system of claim 1, wherein a width of the cradle (302) is less than a length of the cradle and a height of the cradle (302) is greater than the length of the cradle (302).

4. The system of claim 1, wherein a first pad (400) of the plurality of pads (400, 500, 600) has a first color and is shaped to support the head or extremity at a first angle, and a second pad (600) of the plurality of pads (400, 500, 600) has a second color, different from the first color, and is shaped to support the head or extremity at a second angle, different from the first angle.

5. The system of claim 4, wherein the first pad (400) comprises a first pad cradle (402) including first opposing sidewalls (406, 408, 506, 508) and a first body (404, 504), and where the first body (404, 504) is horizontal relative to the base (304) of the cradle (302).

6. The system of claim 4, wherein the second pad (600) comprises a second pad cradle (602) including second opposing sidewalls (606, 608) and a second body (604), wherein the second opposing sidewalls (606, 608) increase in thickness towards the second body (604), and the second body (604) increases in thickness from a first end (612) to a second end (614).

7. The system of claim 5, wherein the first opposing sidewalls (406, 408) and the first body (404) have a similar thickness throughout.

8. The system of claim 5, wherein at least a portion of the first opposing sidewalls (506, 508) have an irregular thickness.

9. The system of claim 1, the opposing sidewalls further comprising a first sidewall (306) and a second sidewall (308) mirroring the first sidewall (306), wherein the first sidewall (306) has a first slot (316a) positioned directly above a second slot (318a), and wherein the first slot (316a) is oriented horizontal relative to the base (304) of the cradle (302) and the second slot (318a) is oriented at an angle relative to the first slot (316a).

10. The system of claim 9, wherein the angle of the second slot (318a) and a tilt angle of a first pad (400) of the plurality of pads (400, 500, 600) are similar.

11. A support assembly for use with an imaging system (100), the support assembly comprising:
a cradle (302) including a base (304) and opposing sidewalls (306, 308); and
a plurality of pads (400, 500, 600) shaped to seat against the cradle (302) within an opening (396) formed between the opposing sidewalls (306, 308), and
where a range of discrete angles are achieved by stacking one or more pads of the plurality of pads (400, 500, 600) in the cradle (302),
where a first pad (400) of the plurality of pads (400, 500, 600) has a first angle relative to the base (304) of the cradle (302), and
where a second pad (600) of the plurality of pads (400, 500, 600) has a second angle (652) relative to the base (304) of the cradle (302).

12. The support assembly of claim 11, wherein the plurality of pads (400, 500, 600) are formed from a foam material.

13. The support assembly of claim 11, wherein the cradle (302) is formed from carbon fiber and an outer surface (330) of the cradle (302) has a fastening material (326) affixed thereto.

14. The support assembly of claim 11, wherein a first fastener (340) is positioned on an inner surface of the cradle (302), a second fastener (454) is positioned on a lower surface of the first pad (400) and the lower surface of the second pad (600), and a third fastener (642) is positioned on the inner surface of the second pad (600).

15. The support assembly of claim 14, wherein face-sharing contact between the first fastener (340) and the second fastener (454) secures the first pad (400) or the second pad (600) to the cradle (302), and face-sharing contact between the second fastener (454) and the third fastener (642) secures the first pad (400) to the second pad (600).
